# EUROPEAN PATENT SPECIFICATION

(11) **EP 3 932 464 B1**
(45) Date of publication and mention of the grant of the patent: **10.09.2025**
(21) Application number: 21192999.7
(22) Date of filing: 18.05.2011
(51) Int. Cl.: A61M 37/00, A61M 39/02, A61M 39/00

(54) **REINFORCED SEPTUM FOR AN IMPLANTABLE MEDICAL DEVICE**
VERSTÄRKTES SEPTUM FÜR EINE IMPLANTIERBARE MEDIZINISCHE VORRICHTUNG
SEPTUM RENFORCÉ POUR UN DISPOSITIF MÉDICAL IMPLANTABLE

(30) Priority: 18.05.2010 US 34584610 P
(43) Date of publication of application: 05.01.2022
(62) Divisional of application: 11784194.0
(73) Proprietor: C. R. Bard, Inc., Franklin Lakes, NJ 07417 (US)
(72) Inventor: HIBDON, Dwight, Park City, UT 84098 (US); CHRISTIAN, Kelly J., Draper, UT 84020 (US); FARNWORTH, Charles L., Riverton, UT 84065 (US)
(74) Representative: Hoffmann Eitle

(56) References cited:
- WO-A1-99/34859
- GB-A- 2 191 701
- JP-A- 2000 079 168
- US-A- 4 405 320
- US-A1- 2006 264 898

## Description

### BACKGROUND

GB 2 191 701 A discloses a self-sealing subcutaneous infusion and withdrawal device or septum including a cup-shaped needle stop member 12 that defines a fluid chamber 44 having a fluid transfer opening. A jacket 26 enveloped the needle stop member and includes an integrally formed delivery tube 30 that aligns with the fluid transfer opening in the needle stop member. The fluid chamber of the needle stop member is sealed by a needle penetrable sealing member to provide a mechanical leak-tight seal.

US 4,405,320 discloses a septum retaining means for an implantable tubular percutaneous device.

US 2006/264898 Al discloses access ports for providing subcutaneous access to a patient. Such access ports may include: at least one structural element configured for resisting deformation of the septum.

JP 2000-079168 A discloses an access port with an opening sealed by a retaining ring.

### BRIEF SUMMARY

In a first aspect of the present invention, there is provided an implantable access port according to claim 1.

Briefly summarized, the present disclosure is directed to a septum for use in sealably covering a fluid cavity of an implantable medical device, such as an access port. The needle-penetrable septum is resilient and includes a reinforcement structure that bolsters septum placement over the fluid cavity so as to inhibit unintended separation of the septum from the medical device when the fluid cavity is under pressure, such as during power injection of fluid into the fluid cavity. The reinforcement structure further assists in preventing septum separation during other septum stress events including insertion of a needle through the septum to access the port fluid cavity, or removal of the needle from the septum when access is no longer needed.

In one embodiment the septum comprises a resilient septum body that includes a flange disposed about a perimeter thereof. A reinforcement component is disposed in the flange for reinforcing engagement of the flange with a corresponding groove defined about an opening to the fluid cavity of the medical device so as to inhibit unintended detachment of the septum from the medical device. The reinforcement component in one embodiment includes an annular cord disposed in the flange.

Various methods for inserting the reinforced septum into the groove in order to sealably cover the fluid cavity are also disclosed, including temporary deformation of the septum, oversizing of the outer diameter of the groove, and the use of memory shape materials for the reinforcement cord. A septum configured as described herein can thus be secured to the access port without the need of an additional cap or retaining ring. This in turn enables an integral access port body of single-piece construction to be employed, which provides simplified design and construction.

These and other features of embodiments of the present disclosure will become more fully apparent from the following description and appended claims, or may be learned by the practice of embodiments of the invention as set forth hereinafter.

### BRIEF DESCRIPTION OF THE DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1 is a perspective view of an access port that serves as one possible environment in which an embodiment of the present disclosure can be practiced;
FIG. 1A is a cross sectional side view of a portion of an implantable access port including a septum configured in accordance with one embodiment;
FIGS. 2A and 2B respectively show top and cross sectional side views of a septum configured in accordance with one embodiment;
FIGS. 3A and 3B respectively show perspective and cross sectional side views of an access port body configured in accordance with one embodiment;
FIGS. 4A and 4B respectively show perspective and cross sectional side views of an access port in accordance with one embodiment;
FIG. 5 is a cross sectional side view of an access port body in accordance with one embodiment;
FIG. 6 is cross sectional side view of an access port including a septum according to one embodiment;
FIG. 7 is a cross sectional side view of an access port including a septum according to another embodiment;
FIG. 8A-8D are cross sectional side views of possible access port groove shapes in accordance with one embodiment;
FIGS. 9A-9C are cross sectional side views showing insertion of a septum into a groove of an access port;
FIG. 10 is a top view of a septum including a segmented cord in accordance with one embodiment; and
FIG. 11 is a cross sectional side view of a portion of an implantable access port including a septum configured in accordance with one embodiment.

### DETAILED DESCRIPTION OF SELECTED EMBODIMENTS

Reference will now be made to figures wherein like structures will be provided with like reference designations. It is understood that the drawings are diagrammatic and schematic representations of exemplary embodiments of the present invention, and are neither limiting nor necessarily drawn to scale.

For clarity it is to be understood that the word "proximal" refers to a direction relatively closer to a clinician using the device to be described herein, while the word "distal" refers to a direction relatively further from the clinician. For example, the end of a transcutaneous catheter placed within the body of a patient is considered a distal end of the catheter, while the catheter end remaining outside the body is a proximal end of the catheter. Also, the words "including," "has," and "having," as used herein, including the claims, shall have the same meaning as the word "comprising."

Embodiments of the present invention are generally directed to a septum for use in sealably covering a reservoir, or fluid cavity, of an implantable medical device, such as an access port. The septum is needle-penetrable so as to enable piercing thereof by a needle or other suitable cannula or device to infuse fluids into, or remove fluids from, the fluid cavity.

In one embodiment, the septum is resilient and includes a reinforcement structure that bolsters septum placement over the fluid cavity so as to prevent blow-out of the septum, *i.e.,* unintended separation of the septum from the medical device when the fluid cavity is under pressure, such as during pressurized injection of fluid into the fluid cavity of an access port. Power injection of fluids into an access port is one example of such pressurized fluid injection, wherein fluid is injected into the fluid cavity of the access port at a rate of about five ml per second at a pressure of about 300 psi, for example. Other fluid pressure scenarios are also possible.

In one embodiment, the reinforcement structure of the septum includes an annular bead, or cord, disposed within a flange circumventing an outer perimeter of the septum. The cord is configured to provide for reinforced retention of the septum flange within a groove defined about an opening of the fluid cavity of the access port such that the septum sealably covers the fluid cavity. So configured, the septum is secured to the access port without the need of an additional cap or retaining ring. Indeed, in one embodiment an integral access port body of single-piece construction can be used, thus providing simplified design and construction.

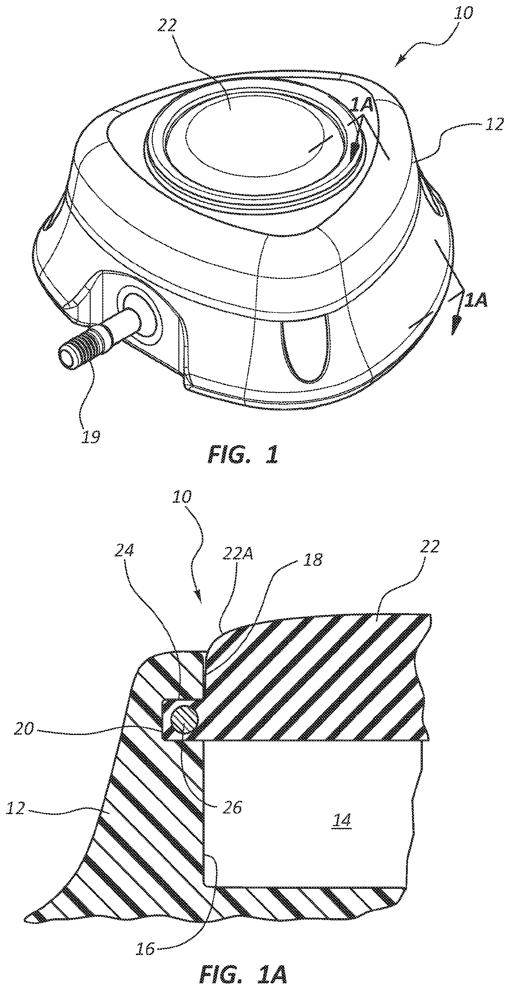

Reference is first made to FIGS. 1 and 1A, which depict various details regarding an implantable access port, generally designated at 10, according to one embodiment. The port 10 includes a body 12 including a suitable material. In one embodiment, the port body 12 includes plastic, such as an acetyl resin for instance, though it is appreciated that other suitable plastics and materials can be employed for the port body, including titanium or other metals or combinations of metals, plastics, or both. The body 12 defines a generally cylindrical fluid cavity 14 (though other cavity shapes are possible) defined in part by a wall 16. A stem 19 in fluid communication with the fluid cavity 14 is also included so that fluids can be passed to and from the fluid cavity.

As best seen in FIG. 1A, the body 12 defines an opening 18 to the fluid cavity 14. An annular recess, or groove 20, is defined in the wall 16 proximate the fluid cavity opening 18. The groove 20 generally defines a rectangular cross sectional shape and further defines an outer diameter ("OD") 20A. Note that other cross sectional shapes are possible for the groove. The groove 20 is sized to receive therein a flange of a septum, described below.

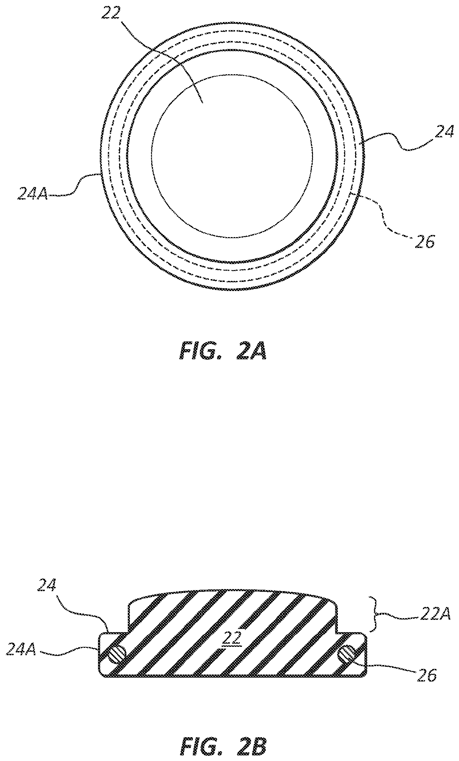

As mentioned, and with additional reference to FIGS. 2A and 2B, the access port 10 further includes a septum 22 disposed in the opening 18 and configured to sealably cover the fluid cavity 14. The septum 22 includes a suitable material such as silicone that enables it to be penetrated by a needle or other cannula so as to provide fluid access to the fluid cavity 14, such as for the infusion into or removal of fluids therefrom. The silicone or other suitable material included in the septum 22 also enables the septum to be resiliently compliant.

As best seen in FIGS. 2A and 2B, the septum 22 in the present embodiment is circular and includes an annular flange 24 extending about a perimeter of the septum. The flange 24 itself defines an outer diameter ("OD") 24A and is sized and configured to be received within the groove 20 such that the septum 22 is disposed in the opening 18 and sealably covers the fluid cavity 14. So disposed, the septum 22 further defines an upper portion 22A disposed above the flange 24, as viewed from the perspective shown in FIG. 1A.

As seen in FIGS. 1A, 2A, and 2B, a reinforcement structure is included in the septum 22. In particular, an annular reinforcement bead, or cord 26, is included within the septum flange 24. Though shown fully encapsulated by the septum flange 24, the reinforcement cord 26 in one embodiment could be partially exposed. It is appreciated that the reinforcement cord 26 can be included within the septum flange 24 as shown in FIGS. 1A, 2A, and 2B via any one of a plurality of methods, including insert molding of the cord into the septum, mechanical insertion into the flange after cutting a circular slit therein followed by re-sealing of the slit, etc. Also, the reinforcement cord shown here includes a round cross sectional profile; it is appreciated, however, that the cord can include other cross sectional profiles, including square, triangular, pentagonal, polygonal, oval, twisted, slotted, spring coiled, etc.

The reinforcement cord 26 is non-resilient along its circular axis such that, when the flange 24 is received within the groove 20 of the fluid cavity 14, the cord provides rigidity to the flange and prevents deformation thereof. This in turn prevents blow-out or unintended detachment of the septum 22 from the port body 12, especially when the port fluid cavity 14 is under pressurization, such as during power injection, for instance. So configured, then, the reinforcement cord reinforces, or bolsters, engagement of the flange with the groove of the port body.

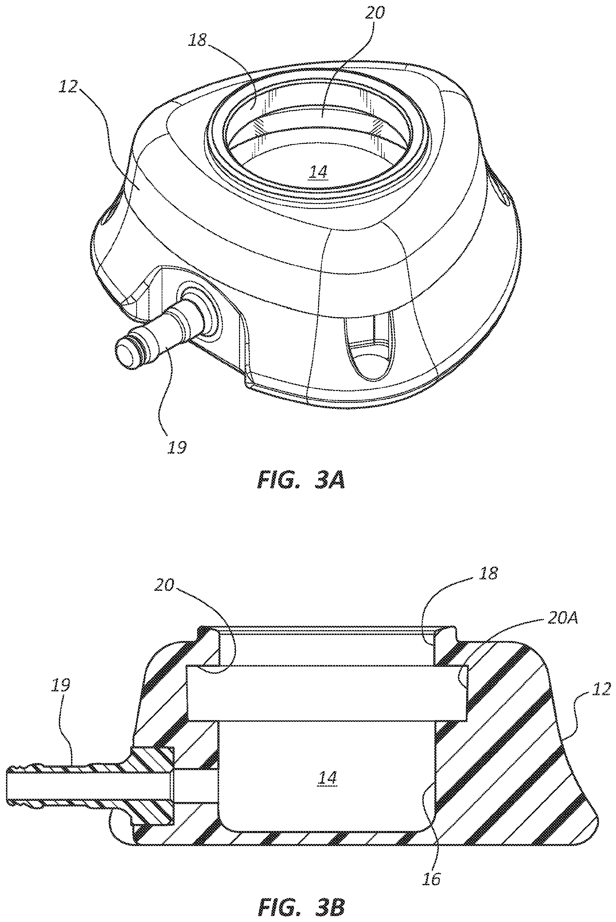

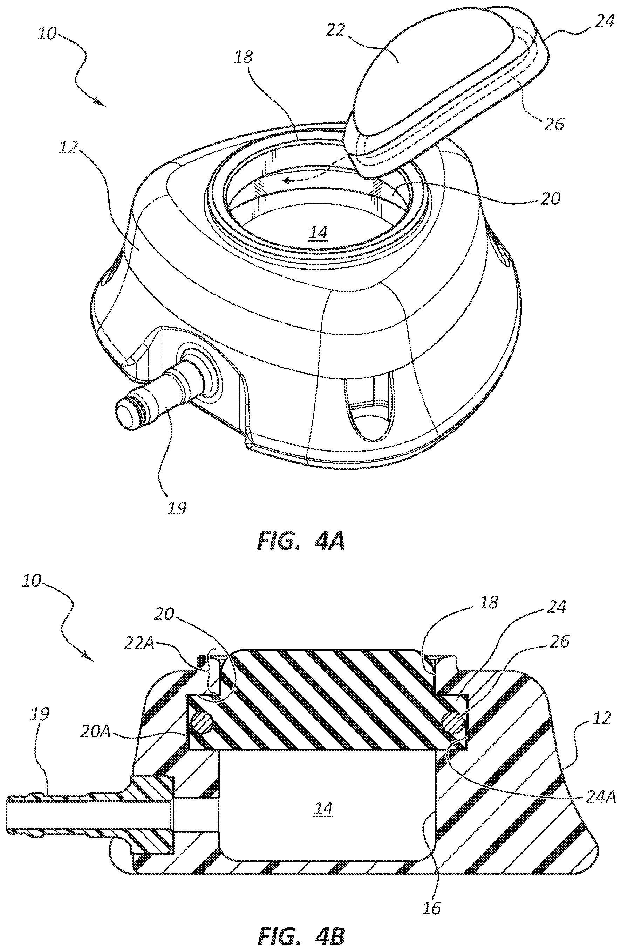

FIGS. 3A and 3B show details of the port body 10 before attachment of the septum 22 thereto. In one embodiment, placement of the septum 22 in the access port 10 can include deforming the septum, such as by partially folding in the manner shown in FIG. 4A. Note that even though it is substantially non-elastic in a direction parallel to its circular axis, in one embodiment the reinforcement cord is capable of a limited amount of deformation in other directions, such as the folding deformation shown in FIG. 4A.

Once partially folded as in FIG. 4A, the septum 22 can then be inserted into the fluid cavity 14 via the opening 18 such that a portion of the reinforced septum flange 24 seats within the groove 20. Portions of the flange 24 adjacent the seated portion can then be worked into the groove 20 until the flange is completely seated therein and the septum 22 is no longer deformed, as in FIG. 4B. In one embodiment, it is appreciated that full seating of the septum flange occurs with the assistance of temporary pressurization and/or depressurization of the fluid cavity.

In another embodiment, the septum can be placed in the fluid cavity via the assistance of a placement tool to temporarily deform the septum so as to insert its periphery into the port body groove. In one embodiment, the tool includes a tube that radially compresses the septum and a plunger that pushes the compressed septum out the end of the tube and into the groove of the port body. In another embodiment, the tool includes a funnel shaped member in which the septum is initially disposed, and further including a rolling rod that can be pressed along the periphery of the septum to pass the septum periphery out of the funnel and into the port body groove. These and other insertion tools are therefore contemplated.

In one embodiment, the reinforcement cord 26 includes a plastic, such as polypropylene, acetyl resin, or other plastic or thermoplastic that includes suitable stiffness so as to prevent buckling under hoop stress. In other embodiments, the cord can include any suitable material such as woven or non-woven metals, plastics, ceramics, high durometer silicones, titanium, stainless steel, nylons, copolymers, and acrylics, etc. In yet another embodiment, the reinforcement cord can include a silicone with a durometer rating that renders it harder relative to a hardness of the septum itself.

In one embodiment the reinforcement cord includes a nickel and titanium alloy commonly known as nitinol, which is a shape memory material. So configured, the reinforcement cord can be initially formed and disposed within the septum while defining a first shape configuration that facilitates relatively simple placement of at least a portion of the septum flange into the groove of the port fluid cavity. Such a first shape configuration of the cord can cause a first half of the septum to assume a partial folded shape while a second half of the septum remains unfolded, for instance. After the unfolded portion of the septum is seated in the groove. the septum can be heated so as to activate the shape memory characteristic of the nitinol reinforcement cord, which causes the cord to change shape to a predetermined second shape configuration. In one embodiment, the second shape configuration is a circularly flat cord configuration, such as that shown in FIGS. 2A and 2B, for instance. Shaping of the reinforcement cord to the second shape configuration causes the formerly folded half of the septum to flatten, which enables the rest of the septum flange to seat into the groove and sealably cover the fluid cavity. Thus, the septum disposed in a final flat configuration with the entire flange is disposed within the groove.

As mentioned, once the septum flange is fully seated within the groove, the reinforcement cord acts as a strengthening element to inhibit unintended removal of the septum from its position covering the fluid cavity of the implantable access port. In addition, in one embodiment the reinforcement cord maintains the septum flange in place within the groove so as to enable construction of the access port body as an integral, single-piece component, *i.e.,* without the need for a retaining ring or cap to retain the septum in place. This in turn simplifies construction and design of the access port.

Note that, though an access port is shown and described herein, in other embodiments other implantable medical devices that include a septum secured thereto can also benefit from the principles of the present disclosure. As such, the present discussion should not be intended to limit the embodiments of the present disclosure in any way.

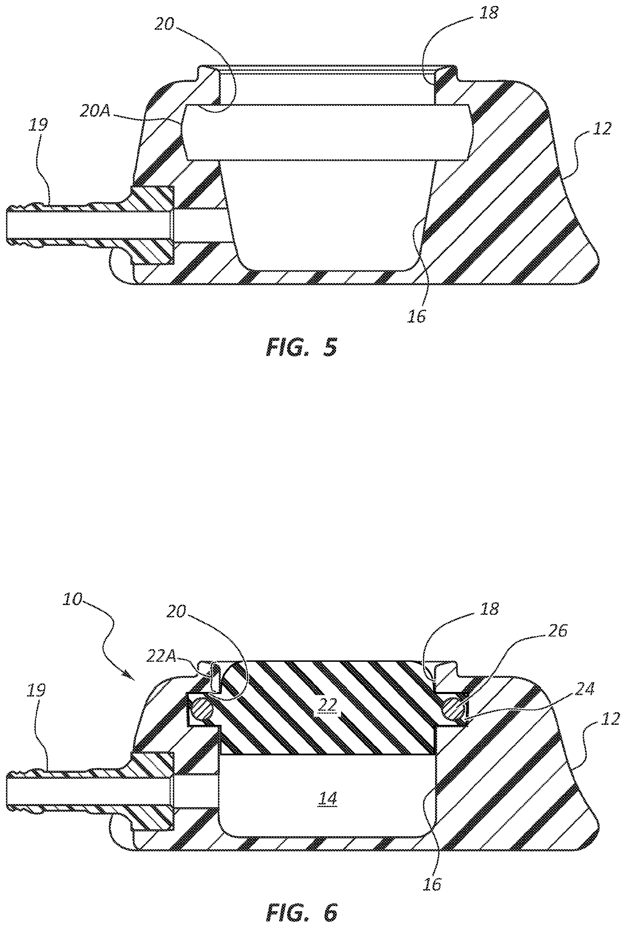

FIG. 5 shows that in one embodiment, the fluid cavity can be shaped other than cylindrically. For example the side wall 16 of the port body 12 in FIG. 5 is slanted so as to define a frustoconically shaped fluid cavity 14. This configuration can be employed to assist in urging the septum flange 24 to seat in the groove 20. Other fluid cavity shapes are also possible. More generally, it is appreciated that the top view cross sectional shapes of the septum and fluid cavity can also vary from circular, including oval shapes for instance.

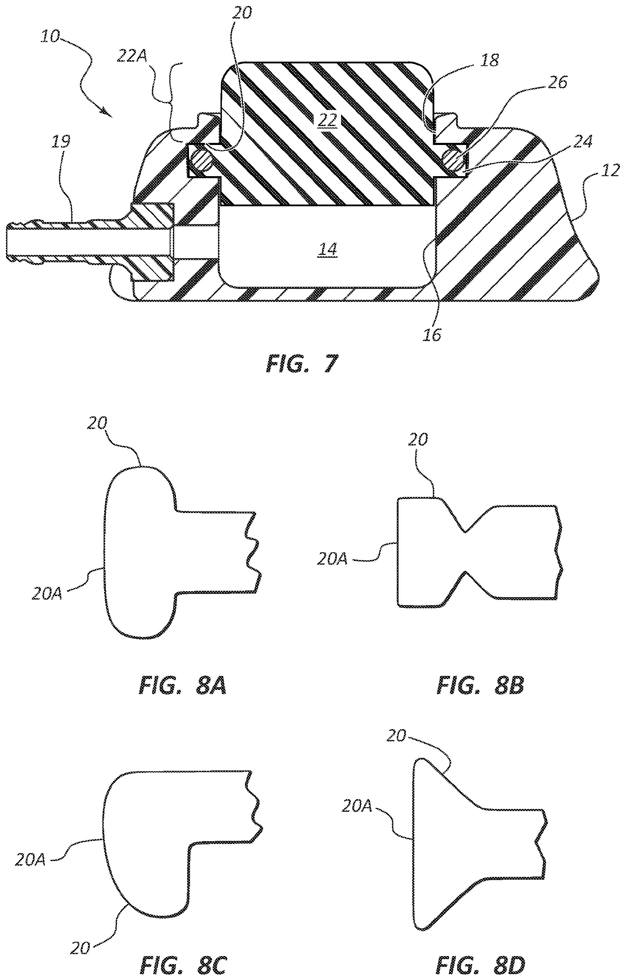

As mentioned further above, note that the cross sectional shape of the groove can vary from the rectangular configuration shown in FIG. 1A. For instance, in one embodiment the OD 20A of the groove 20 can define a concave shape. In another embodiment, the groove can include a convex, or cupped, shape as shown in FIG. 5 so as to assist in retaining the septum flange. In one embodiment, the OD 24A of the flange 24 can be correspondingly shaped to match the groove OD 20A. Further non-limiting examples of possible cross sectional shapes for the groove 20 are shown in FIGS. 8A-8D.

It is appreciated that the position of the groove in the fluid cavity can vary according to design. Moreover, the thickness of the groove and corresponding septum flange can also vary from what is shown and described herein. These and other variations are therefore contemplated.

FIGS. 6 and 7 show that the size of the septum 22 can vary from thin (FIG. 6), including a relatively thin septum upper portion 22A to thick (FIG. 7), wherein the septum upper portion is relatively thick. In the case of the thin septum 22 of FIG. 6, an area of radial compression includes a majority of the thickness of the septum, while the thick septum of FIG. 7 includes only a lower portion of the septum thickness where radial compression is present. Note that the cord-equipped septum discussed herein enables a septum to be mated to the access port body without unduly increasing the thickness of the port, thus preserving a low profile port configuration, if desired.

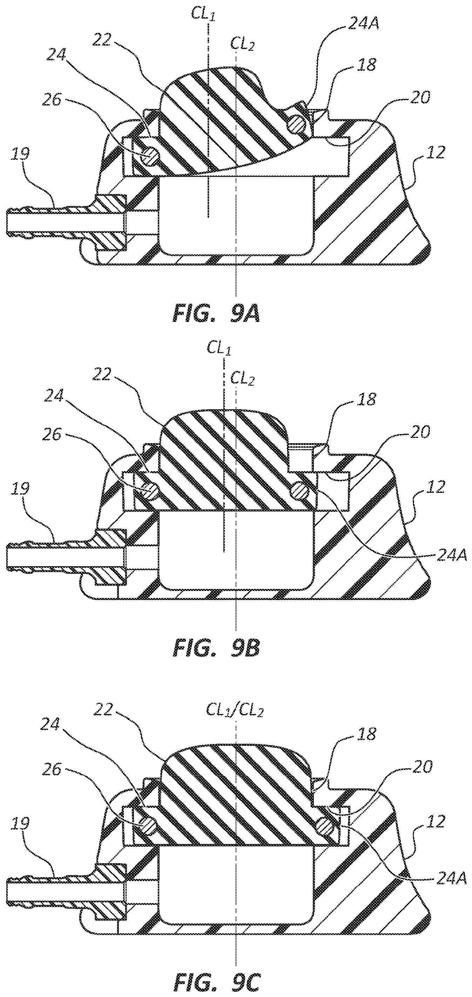

FIGS. 9A-9C show that, in one embodiment, the OD 20A of the groove 20 can be greater relative to the OD 24A of the septum flange 24 such that ease of insertion of the septum flange into the groove is facilitated. In particular, these figures show a process for inserting the septum into the port body including laterally compressing the septum 22 such that its centerline CL₁ is offset from the centerline CL₂ of the port body 12 while inserting a portion of the septum flange 24 into the groove 20. With the septum still laterally compressed, the opposing portion of the flange 24 is inserted into the groove 20. Such insertion is made possible due to the oversized nature of the groove OD 20A. Once the flange 24 has been seated in the groove 20, compression of the septum 22 is released, and the septum centers in the opening 18 such that is centerline CL₁ substantially aligns with the centerline CL₂ of the port body 12.

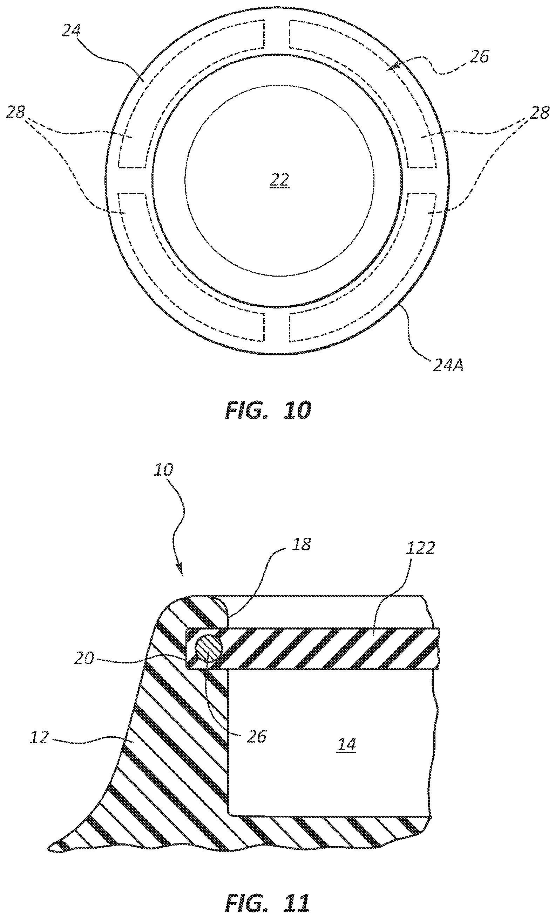

FIG. 10 shows that in one embodiment the reinforcement cord can include other than a continuous configuration. Indeed, FIG. 10 shows the reinforcement cord as including a plurality of reinforcement segments 28 that are arranged in a generally circular pattern and disposed within the flange 24 of the septum 22. These and other reinforcement schemes are therefore contemplated. In another embodiment, a plurality of reinforcement cords can be included in the septum flange. FIG. 11 shows that, in one embodiment, a septum 122 that includes no upper portion can also include the reinforcement cord 26, thus illustrating the ability to include a reinforcement cord in septa of varying types and configurations.

The described embodiments are to be considered in all respects only as illustrative, not restrictive. The scope of the embodiments is, therefore, indicated by the appended claims rather than by the foregoing description. All changes that come within the meaning and range of equivalency of the claims are to be embraced within their scope.

## Claims

1. An implantable access port (10), comprising:
a body (12) defining a fluid cavity (14) and a recess (20); and
a needle-penetrable septum (22) covering the fluid cavity (14), the septum including:
a flange (24) that is received within the recess of the port body; and
a reinforcement structure (26) disposed within the flange,
**characterised in that**
the body (12) of the access port is an integral, single-piece construction.

2. The access port defined in claim 1, wherein the port (10) includes at least one of a thermoplastic and a metal, wherein the recess (20) includes an annular groove defined proximate an opening of the fluid cavity, and wherein the flange (24) is annularly defined about the septum.

3. The access port as defined in claim 1 or 2, wherein the reinforcement structure (26) includes an annular cord disposed within the flange (24) of the septum (22) so as to prevent removal of the flange from the recess when the access port is subjected to power injection.

4. The access port as defined in claim 3, wherein the septum (22) is substantially circular and includes silicone, and wherein the reinforcement cord (26) is insert-molded within the flange (24) of the septum (22).

5. The access port as defined in claim 3 or 4, wherein the reinforcement cord (26) includes at least one of polypropylene, acetyl resin, silicone, titanium, and stainless steel.

6. The access port as defined in any preceding claim, wherein the reinforcement structure (26) bolsters engagement of the flange (24) with the recess (20).

7. The access port as defined in any preceding claim, wherein the body (12) defines an opening (18) to the fluid cavity (14).

8. The access port as defined in claim 7, wherein the recess (20) includes an annular groove defined in the access port body (12) between the fluid cavity (14) and the opening (18), the annular groove having a diameter greater than a diameter of the opening and a diameter of the fluid cavity.

9. The access port as defined in claim 7 or 8, wherein the needle-penetrable septum (22) comprises an upper portion (22A) disposed in the opening.

10. The access port as defined in claim 9, wherein the flange (24) is an annular flange disposed in the recess (20), the annular flange extending radially outward from a lower portion and having a diameter greater than a diameter of the upper portion.

11. The access port of any preceding claim, wherein the reinforcement structure (26) is an annular reinforcement structure and the flange (24) is an annular flange.

12. The access port of claim 11, wherein the diameter of the annular groove (20) is greater than the diameter of the annular flange (24), and/or wherein the annular reinforcement structure (26) includes an annular cord formed from a shape-memory material.

13. The access port of claim 11 or 12, wherein the annular reinforcement structure (26) includes a plurality of discrete segments.

14. The access port of claim 11 or 12, wherein the annular reinforcement structure (26) is a continuous annular cord including a circular cross-sectional shape, and is substantially non-resilient along an annular axis thereof,
wherein, optionally, the continuous annular cord comprises a memory shape material and is capable of assuming a first shape configuration during placement of the annular flange in the groove, and a second shape configuration after at least partial placement of the annular flange in the groove.

## Patentansprüche

1. Implantierbarer Zugangsanschluss (10), umfassend:
einen Körper (12), der einen Fluidhohlraum (14) und eine Aussparung (20) definiert; und
ein von einer Nadel durchdringbares Septum (22), das den Fluidhohlraum (14) abdeckt, wobei das Septum einschließt:
einen Flansch (24), der in der Aussparung des Anschlusskörpers aufgenommen ist; und
eine Verstärkungsstruktur (26), die in dem Flansch angeordnet ist,
**dadurch gekennzeichnet, dass**
der Körper (12) des Zugangsanschlusses eine integrierte, einstückige Konstruktion ist.

2. Zugangsanschluss nach Anspruch 1, wobei der Anschluss (10) mindestens eines von einem Thermoplast und einem Metall einschließt, wobei die Aussparung (20) eine ringförmige Rille einschließt, die in der Nähe einer Öffnung des Fluidhohlraums definiert ist, und wobei der Flansch (24) ringförmig um das Septum herum definiert ist.

3. Zugangsanschluss nach Anspruch 1 oder 2, wobei die Verstärkungsstruktur (26) einen ringförmigen Strang einschließt, der in dem Flansch (24) des Septums (22) angeordnet ist, um eine Entfernung des Flanschs aus der Aussparung zu verhindern, wenn der Zugangsanschluss einer energiegetriebenen Injektion unterzogen wird.

4. Zugangsanschluss nach Anspruch 3, wobei das Septum (22) im Wesentlichen kreisförmig ist und Silikon einschließt, und wobei der Verstärkungsstrang (26) mittels Inserttechnik in dem Flansch (24) des Septums (22) angeordnet ist.

5. Zugangsanschluss nach Anspruch 3 oder 4, wobei der Verstärkungsstrang (26) mindestens eines von Polypropylen, Acetylharz, Silikon, Titan und Edelstahl einschließt.

6. Zugangsanschluss nach einem vorstehenden Anspruch, wobei die Verstärkungsstruktur (26) einen Eingriff des Flanschs (24) mit der Aussparung (20) unterstützt.

7. Zugangsanschluss nach einem vorstehenden Anspruch, wobei der Körper (12) eine Öffnung (18) in den Fluidhohlraum (14) definiert.

8. Zugangsanschluss nach Anspruch 7, wobei die Aussparung (20) eine ringförmige Rille einschließt, die in dem Körper (12) des Zugangsanschlusses zwischen dem Fluidhohlraum (14) und der Öffnung (18) definiert ist, wobei die ringförmige Rille einen Durchmesser aufweist, der größer als ein Durchmesser der Öffnung und ein Durchmesser des Fluidhohlraums ist.

9. Zugangsanschluss nach Anspruch 7 oder 8, wobei das von einer Nadel durchdringbare Septum (22) einen oberen Abschnitt (22A) umfasst, der in der Öffnung angeordnet ist.

10. Zugangsanschluss nach Anspruch 9, wobei der Flansch (24) ein ringförmiger Flansch ist, der in der Aussparung (20) angeordnet ist, wobei sich der ringförmige Flansch von einem unteren Abschnitt radial nach außen erstreckt und einen Durchmesser aufweist, der größer als ein Durchmesser des oberen Abschnitts ist.

11. Zugangsanschluss nach einem vorstehenden Anspruch, wobei die Verstärkungsstruktur (26) eine ringförmige Verstärkungsstruktur ist und der Flansch (24) ein ringförmiger Flansch ist.

12. Zugangsanschluss nach Anspruch 11, wobei der Durchmesser der ringförmigen Rille (20) größer als der Durchmesser des ringförmigen Flanschs (24) ist, und/oder wobei die ringförmige Verstärkungsstruktur (26) einen ringförmigen Strang einschließt, der aus einem Formgedächtnismaterial gebildet ist.

13. Zugangsanschluss nach Anspruch 11 oder 12, wobei die ringförmige Verstärkungsstruktur (26) eine Mehrzahl von individuellen Segmenten einschließt.

14. Zugangsanschluss nach Anspruch 11 oder 12, wobei die ringförmige Verstärkungsstruktur (26) ein durchgängiger ringförmiger Strang ist, der eine kreisförmige Querschnittsform einschließt und entlang einer ringförmigen Achse davon im Wesentlichen nicht nachgiebig ist,
wobei optional der durchgängige ringförmige Strang ein Formgedächtnismaterial umfasst und in der Lage ist, eine erste Formkonfiguration während einer Platzierung des ringförmigen Flanschs in der Rille und eine zweite Formkonfiguration nach einer zumindest teilweisen Platzierung des ringförmigen Flanschs in der Rille anzunehmen.

## Revendications

1. Orifice (10) d'accès implantable, comprenant :
un corps (12) définissant une cavité fluidique (14) et un évidement (20) ; et
un septum (22) pénétrable par aiguille recouvrant la cavité fluidique (14), le septum incluant :
une bride (24) qui est reçue à l'intérieur de l'évidement du corps d'orifice ; et
une structure (26) de renfort disposée à l'intérieur de la bride, **caractérisé en ce que** le corps (12) de l'orifice d'accès est une construction monobloc intégrale.

2. Orifice d'accès selon la revendication 1, dans lequel l'orifice (10) inclut au moins l'un parmi un thermoplastique et un métal, dans lequel l'évidement (20) inclut une rainure annulaire définie à proximité d'une ouverture de la cavité fluidique, et dans lequel la bride (24) est définie annulairement autour du septum.

3. Orifice d'accès selon la revendication 1 ou la revendication 2, dans lequel la structure (26) de renfort inclut un cordon annulaire disposé à l'intérieur de la bride (24) du septum (22) afin d'empêcher le retrait de la bride de l'évidement lorsque l'orifice d'accès est soumis à une injection de puissance.

4. Orifice d'accès selon la revendication 3, dans lequel le septum (22) est sensiblement circulaire et inclut du silicone, et dans lequel le cordon (26) de renfort est moulé par insertion à l'intérieur de la bride (24) du septum (22).

5. Orifice d'accès selon la revendication 3 ou la revendication 4, dans lequel le cordon (26) de renfort inclut au moins l'un parmi le polypropylène, la résine d'acétyle, le silicone, le titane et l'acier inoxydable.

6. Orifice d'accès selon une quelconque revendication précédente, dans lequel la structure (26) de renfort renforce l'engagement de la bride (24) avec l'évidement (20).

7. Orifice d'accès selon une quelconque revendication précédente, dans lequel le corps (12) définit une ouverture (18) vers la cavité fluidique (14).

8. Orifice d'accès selon la revendication 7, dans lequel l'évidement (20) inclut une rainure annulaire définie dans le corps (12) d'orifice d'accès entre la cavité fluidique (14) et l'ouverture (18), la rainure annulaire présentant un diamètre supérieur à un diamètre de l'ouverture et un diamètre de la cavité fluidique.

9. Orifice d'accès selon la revendication 7 ou la revendication 8, dans lequel le septum (22) pénétrable par aiguille comprend une partie supérieure (22A) disposée dans l'ouverture.

10. Orifice d'accès selon la revendication 9, dans lequel la bride (24) est une bride annulaire disposée dans l'évidement (20), la bride annulaire s'étendant radialement vers l'extérieur à partir d'une partie inférieure et présentant un diamètre supérieur à un diamètre de la partie supérieure.

11. Orifice d'accès selon une quelconque revendication précédente, dans lequel la structure (26) de renfort est une structure de renfort annulaire et la bride (24) est une bride annulaire.

12. Orifice d'accès selon la revendication 11, dans lequel le diamètre de la rainure annulaire (20) est supérieur au diamètre de la bride (24) annulaire, et/ou dans lequel la structure (26) de renfort annulaire inclut un cordon annulaire formé à partir d'un matériau à mémoire de forme.

13. Orifice d'accès selon la revendication 11 ou la revendication 12, dans lequel la structure (26) de renfort annulaire inclut une pluralité de segments discrets.

14. Orifice d'accès selon la revendication 11 ou la revendication 12, dans lequel la structure (26) de renfort annulaire est un cordon annulaire continu incluant une forme en section transversale circulaire, et est sensiblement non élastique le long d'un axe annulaire de celle-ci,
dans lequel, facultativement, le cordon annulaire continu comprend un matériau à mémoire de forme et est capable de prendre une première configuration de forme lors du placement de la bride annulaire dans la rainure, et une deuxième configuration de forme après un placement au moins partiel de la bride annulaire dans la rainure.
